# EUROPEAN PATENT SPECIFICATION

(11) **EP 1 550 669 B1**
(45) Date of publication and mention of the grant of the patent: **06.08.2008**
(21) Application number: 04030315.8
(22) Date of filing: 15.12.2003
(51) Int. Cl.: C07K 1/113, C07K 14/72

(54) **Refolded membrane protein in monodisperse form**
Rückgefaltetes Membranprotein in monodisperser Form
Protéine de la membrane repliée dans une forme monodisperse

(43) Date of publication of application: 06.07.2005
(62) Divisional of application: 03028803.9
(73) Proprietor: M-Phasys GmbH, 72070 Tübingen (DE)
(72) Inventor: Linden, Lars, Dr., 72076 Tübingen (DE); Prytulla, Stefan, Dr., 72127 Kusterdingen (DE); Ostermann, Thomas, Dr., 72070 Unterjesingen (DE); Bähner, Monika, Dr., 72076 Tübingen (DE); Roos, Tilmann, Dr., 72127 Kusterdingen (DE); Thess, Andreas, Dr., 72127 Kusterdingen (DE); Kiefer, Hans, Dr., 72070 Tübingen (DE); Vogt, Wolfgang, Dr., 72072 Tübingen (DE)
(74) Representative: Findeisen, Marco

(56) References cited:
- WO-A-01/14407
- KIEFER H: "In vitro folding of alpha-helical membrane proteins" BIOCHIMICA ET BIOPHYSICA ACTA. BIOMEMBRANES, AMSTERDAM, NL, vol. 1610, no. 1, 17 February 2003 (2003-02-17), pages 57-62, XP004409757 ISSN: 0005-2736
- OKADA T ET AL: "X-RAY DIFFRACTION ANALYSIS OF THREE-DIMENSIONAL CRYSTALS OF BOVINE RHODOPSIN OBTAINED FROM MIXED MICELLES" JOURNAL OF STRUCTURAL BIOLOGY, ORLANDO, US, vol. 130, no. 1, May 2000 (2000-05), pages 73-80, XP001055972 ISSN: 1047-8477
- OSTERMEIER C ET AL: "CRYSTALLIZATION OF MEMBRANE PROTEINS" CURRENT OPINION IN STRUCTURAL BIOLOGY, CURRENT BIOLOGY LTD., LONDON, GB, vol. 7, no. 5, October 1997 (1997-10), pages 697-701, XP001056803 ISSN: 0959-440X

## Description

The present invention relates to a method for preparing a solution of a refolded, recombinantly expressed or chemically synthesized eukaryotic membrane protein in monodisperse form, and to methods for preparing a crystalline form of a recombinantly expressed or chemically synthesized membrane protein.

Methods of these kinds are generally known in the art.

Membrane proteins are of great pharmacological interest because of their importance as therapeutic and/or diagnostic targets. The most important exponents of membrane proteins are G-protein-coupled receptors (GPCRs), ion channels, and transport proteins.

Since the human genome has been deciphered the existence of about 350 GPCRs is known, without including the olfactory and gustatory receptors. The GPCRs analyzed in detail so far are almost exclusively very important pharmacological targets. For example, the nociceptin receptor is of great relevance in pain therapy. There are many companies doing research on antagonists against the nociceptin receptor in order to develop a potent analgesic. Comparable can be said for what concerns the opiate receptors.

Another receptor which is very well examined concerns the β-adrenergic receptor. Antagonists against that receptor are responsible for the regulation of blood pressure.

Many cytokine receptors play an important role in connection with inflammations and allergies. In the middle of interests herewith is the CXCR1 receptor.

Another not very well analyzed group of receptors are the so-called frizzled receptors. To that group belong 10 receptors which inter alia are involved in embryogenesis.

Also the rhodopsin which is located in the eye belongs to the GPCRs in the broadest sense, with rhodopsin being the only membrane receptor that naturally occurs in large amounts. All other receptors are only present in small amounts in the cell membrane.

Dysfunctional ion channels have been described in connection with many diseases, such as cystic fibrosis, diabetes mellitus, myotonia, epilepsy, and others.

Because of the enormous pharmaceutical importance of the membrane proteins there is a need for the finding of substances which are able to interact with membrane proteins, e.g. for their usage as an inhibitor or activator of a GPCR.

By the known high-throughput-screening (HTS) methods it is not possible to find agonists, antagonists or inverse-agonists for/against membrane proteins, e.g. GPCRs, in a satisfactory, time-saving and effective manner. The possible variations with chemical compounds are about 10⁶⁰. Via the current HTS methods it is impossible to use that available chemical space. This is verified by the poor amount of registered drugs in the last years which are based on new innovative structures.

By means of new methods like the so-called *in silico-*screening or the "rational drug design" (RDD) it is possible to find new ligands for membrane proteins more efficiently and faster than with the HTS methods.

The prerequisite for the performance of an *in silico-*screening or RDD is the availability of the 3D-structure of the protein and thus of large amounts of such membrane protein in monodisperse form. In this context the term "monodisperse" is to be understood in the sense of a condition of a substance, e.g. a membrane protein, which is given if said substance has been purified, having a uniform particle size and being dissolved in an appropriate solvent. Furthermore, in order to provide especially well-suited starting material for a crystallization process the protein should be correctly folded without having any post-translational modifications.

What concerns soluble, non-membrane proteins it is very often possible to obtain a solution of large amounts of monodisperse protein which subsequently can be crystallized in order to elucidate their three-dimensional structures. In fact, today the crystallization of soluble proteins is more or less a standard procedure.

More or less the same applies for bacterial membrane proteins which can be natively expressed, purified and, sometimes, even crystallized; cf. Ostermeier C., and Michel H. (1997), "Crystallization of membrane proteins", Current Opinion in Structural Biology, 7: 697 - 701.

The situation is totally different with eukaryotic membrane proteins, such as GPCRs. In the case of producing the membrane protein by expressing it as a recombinant protein, e.g. in *E. coli* bacteria, one usually will obtain the membrane protein in form of so-called inclusion bodies. Those inclusion bodies are aggregates of unsoluble, non- or misfolded membrane protein. Only a small fraction will be obtained as natively folded protein and/or will even be present as a solution of monodisperse protein. Therefore, by usage of common methods, the yield of monodisperse membrane proteins is very small. The problems concerning the purification of membrane proteins for crystallization purposes are e.g. described in Ostermeier C., and Michel H., loc.cit.

A method for preparing a part of a special kind of membrane protein, namely of cytochrome P450 monooxygenase, is described in the WO 03/035693, whereby said part being localized beyond the membrane and is, therefore, actually not a membrane protein. In Buchanan S. K. (1999), "β-barrel proteins from bacterial outer membranes: structure, function and refolding", 9: 455 - 461, also a preparation of a very special kind of β-barrel proteins is described. These membrane proteins are very stable because of their high content of β-pleated sheets. However, the methods disclosed in both documents cannot be transferred to the preparation of other kinds of widespread membrane proteins, such as GPCRs or ion channels because of their high portions of α-helical sections.

In Urbani A., (2001), "Properties of detergent solubilized cytochrome c oxidase (cytochrome cbb3) purified from Pseudomonas stutzeri", FEBS Letters 508: 29 - 35, a method for preparing a special kind of a bacterial membrane protein is disclosed. That known method has been adapted for production of small amounts of bacterial membrane protein cbb₃ and cannot be used for preparation of the most pharmacological interesting human membrane proteins, e.g. GPCRs, especially not for preparation of large amounts of membrane proteins sufficient for subsequent crystallization since in the known method the protein was selectively extracted from bovine retinal membranes.

Okada T. et al. (2000), "X-ray diffraction analysis of three-dimensional crystals of bovine rhodopsin obtained from mixed micelles", Journal of Structural Biology 130: 73-80, disclose a method for preparation of the photoreceptor membrane protein rhodopsin. According to this known method the protein was selectively extracted from bovine retinal membranes and afterwards crystallized. This method is adjusted to a special kind of protein which is the only known GPCR being present in large amounts embedded in the retina. However, all other known GPCRs only occur in very small amounts within biological membranes. Therefore, that known method is not helpful for producing large amounts of most of the interesting GPCRs.

Document DE 199 39 246 A1 discloses a method by which large amounts of different kinds of membrane proteins folded into their native or active structure can be produced. With that method a membrane protein is provided solubilized in a first detergent which is then changed for a second detergent. Herewith, the further above mentioned problem concerning the inclusion bodies problem is solved via a refolding procedure. A problem with that known method is that no solution of refolded membrane protein in monodisperse form is obtained, and that the membrane proteins are not present in a sufficiently homogenous form.

Therefore, it is an object of the present invention to provide a reliable method by which a solution of large amounts of refolded eukaryotic membrane protein in monodisperse form can be obtained and which is also simple to handle. Furthermore, the new method should not be limited to the preparation of a special kind of membrane protein but should also be applicable to widespread membrane proteins such as GPCRs. Moreover, the method should use bacterial expression systems in order to provide sufficient amounts of membrane protein.

According to the present invention, this object is achieved by providing a method for preparing a solution of a refolded, recombinantly expressed or chemically synthesized eukaryotic membrane protein in monodisperse form, consisting of the steps: (a) providing said membrane protein in non-correctly folded form and solubilized in a first detergent, (b) inducing refolding of said membrane protein into its native or active form, and (c) performing a size exclusion chromatography on said solution of refolded membrane protein.

Herewith the problem underlying the present invention is totally solved.

The inventors have realized that with that method it is ensured that the provided membrane protein will be obtained in monodisperse form. It was especially surprising that by means of steps (a) to (c) a homogenous solution of membrane protein being refolded into its active form is yielded. One would rather expect that additional measures had to be carried out for separating unwanted non-active protein from the desired active protein. However, according to the invention the only step needed further to refolding is a size exclusion chromatography according to step (c), e.g. by the use of a Superdex 200 column, by which step protein of a homogeneous size or shape, e.g. monomeric protein, is separated from protein of a size different to that, e.g. from non-monomeric protein. By this size exclusion chromatography also dimeric protein can be separated e.g. from mono-, tri-, tetra-, penta-, hexameric etc. protein. In the case of ion channel proteins which, in most cases, consist of several subunits, it is mostly even desirable to separate the fully assembled channel, e.g. the tetrameric or pentameric protein, from individual subunits or partially assembled ion channels.

A further advantage of that method is that it is not necessary to isolate the interesting protein out of a biological membrane which would require to provide large amounts of biological raw material as starting material, even though the yield of membrane protein would still be scanty. In contrast, by the usage of recombinantly expressed or chemically synthesized protein well-established molecular expression systems and chemical synthesis methods, respectively, can be applied which are capable of producing sufficient amounts of membrane protein for, e.g., a subsequent crystallization.

The membrane proteins yielded according to the invention are sufficiently homogenous and monodisperse, e.g. for a subsequent usage in a crystallization procedure for elucidating their three dimensional structure.

Furthermore, by means of the inventive method also widespread and pharmacological important eukaryotic membrane proteins such as GPCRs can be obtained in large amounts, i.e. the method is not limited to the preparation of untypical and less interesting kinds of e.g. bacterial membrane proteins or β-barrel proteins.

The generic term "size exclusion chromatography" comprises all physico-chemical separation methods by which substances, e.g. membrane proteins, can be separated from each other on account of their different sizes. Examples for those chromatographic methods are filtration, gel filtration/chromatograpy, and other techniques well known in the art.

According to the invention it is preferred if between steps (a) and (b) a further step (a') is performed by which a lipid is added to said membrane protein solution.

The inventors have found out that due to the addition of a lipid into the solution containing the first detergent the refolding process is favored and the long-term stability of the refolded protein is improved, i.e. the lipid stabilizes the functional conformation of the membrane protein.

With the new method it is preferred if step (b) comprises step (b') by which said first detergent is exchanged for a second detergent.

This measure has the advantage that on account of the exchange of the detergents, e.g. a strong denaturating first detergent for a mild second detergent, the solubilized membrane protein which possibly originates from an inclusion bodies preparation, can be efficiently transferred into its native or active form. This procedure is described in more detail in DE 199 39 246 A1 the content whereof is herewith incorporated in this application by reference.

An alternative procedure according to the invention relates to step (b) comprising step (b'') by which said first detergent is diluted to an adequately low concentration.

This measure also ensures an efficient transfer of the solubilized denaturated membrane protein into its native or active form, whereas herewith e.g. the usage of a second detergent for inducing the refolding procedure is dispensable.

With the new method it is preferred if said membrane protein is selected from the group consisting of: receptors, preferably from the family of G-protein-coupled receptors (GPCRs), ion channels, transport proteins as well as partial sequences, homologous sequences, mutated sequences and derived sequences of aforementioned group members, whereby it is further preferred if said membrane protein is a mammalian, e.g. a human protein.

This measure has the advantage that herewith the pharmacologically most important membrane proteins being involved in several diseases will be covered. It is now possible for the first time by means of the method according to the invention to prepare large amounts of synthetically and/or recombinantly produced active GPCRs.

According to a preferred embodiment said membrane protein is provided as a histidine-tagged fusion protein.

An expressed histidine-tagged membrane protein has the advantage that it can be purified simply by metal chelating chromatography, for example by usage of a NiNTA column. Furthermore, the so tagged protein can even be purified in denatured state. The preparation, purification and handling of histidine-tagged fusion proteins is well-known and well-established in the art.

According to a further preferred measure said membrane protein is provided in form of inclusion bodies, preferably as a bacterially expressed protein, more preferably as an *E*. *coli* expressed protein.

The provision of the interesting membrane protein in form of inclusion bodies has several advantages. Inclusion bodies are insoluble protein aggregates consisting of biologically inactive and not correctly folded protein. Inclusion bodies are relatively homogenous and purified for what concerns the contained protein, and can be handled in a simple way and e.g. further purified. Moreover, inclusion bodies contain large amount of protein which can be subjected to a refolding process. By the usage of a bacterial or of the *E*. *coli* expression system a technically mature tool is applied which is well-established in most molecular biological laboratories. In addition, the above-mentioned insolubility problem concerning the inclusion bodies is hereby managed in a simple manner, namely by refolding the aggregated protein as in step (b).

It is also preferred if said membrane protein is provided in form of inclusion bodies being synthesized by means of a cell-free expression system, preferably of the Rapid Translation System (RTS).

By this measure a suitable cell-free *in vitro* expression system, e.g. the so-called "Rapid Translation System" (RTS, Roche Diagnostics) is used by which up to 5 mg of the desired protein can be synthesized within 24 hours. The usage of RTS is especially advantageous since it produces much more recombinant membrane protein than traditional cellular expression systems.

With the method according to the invention it is preferred if said added lipid is selected from the group consisting of: naturally extracted phospholipids and synthetic phospholipids; especially brain polar lipid extract, phosphatidyl choline, phosphatidyl ethanolamine, cholesterol, phospholipid, ergosterol, asolectin, sphingomyelin, DOPA. Preferably, said lipid is added in step (b) to a final concentration of about 0,01 to 5 mg/ml, more preferably of about 0,05 to 2 mg/ml, and even more preferably of about 1 mg/ml.

As the inventors have realized, best results in preparating refolded monodisperse membrane protein will be obtained if one of the afore-listed lipids is used. The phosphocholine can originate from soybean or hens' egg, the phospholipid can originate from soybean, brain polar lipid extract can originate from pork, and phospatidyl ethanolamine can originate from sheep brain. Furthermore, the inventors have ascertained that said indicated concentrations will further optimize the yield.

According to the invention it is also preferred if said first detergent is selected from the group consisting of: FOS-choline-8 (N-octylphosphocholine), FOS-choline-9 (N-nonylphosphocholine), FOS-choline-10 (N-decylphosphocholine), FOS-choline-11 (N-undecylphosphocholine), FOS-choline-12 (N-dodecylphosphocholine), FOS-choline-13 (N-tridecylphosphocholine), FOS-choline-14 (N-tetradecylphosphocholine), FOS-choline-15 (N-pentadecylphosphocholine), FOS-choline-16 (N-hexadecylphosphocholine), and N-laroyl-sarcosine. Preferably, said first detergent is provided in a final concentration of about 0,1 to 5, more preferably of about 0,5 to 4, furthermore preferably of about 1 % (w/v).

The inventors have surprisingly found out that the usage of such harsh detergents is advantageous in order to effectively obtain a monodisperse protein preparation. With the indicated concentrations it is ensured that the provided membrane protein in step (a) will be completely unfolded, in order to transfer it into an active and monodisperse form in step (b) and (c). The detergents can e.g. be obtained at Anatrace Inc., Maumee, USA.

It is further preferred if in step (b) additionally SDS and/or urea is added.

By the addition of SDS (sodium dodecyl sulfate), as a strong synthetically anionic detergent, and/or of urea (carbamide), as another strong detergent, it will be ensured that contaminating protein, e.g. thrombin which possibly was used in order to cleave off a fusion part of the protein, is removed. Of course, the SDS and/or urea can subsequently be removed for allowing the membrane protein adopting its native or active form.

With the new method it is also preferred if said second detergent is selected from the group consisting of: maltosides; alkyl phosphocholines having a chain length of C8 to C16; bile acids and derivatives; alkyl-N,N-dimethyl glycin (alkyl=C8 to C16); alkyl glycosides (alkyl=C5 to C12); glucamides; saccharide fatty acid esters. Furthermore, it is preferred, if said second detergent is provided in a final concentration of about 0,01 to 5, preferably of about 0,05 to 1, more preferably of about 0,1 % (w/v).

The inventors have surprisingly realized that by the usage of those largely mild detergents the solubilized proteins will be subjected to a refolding process leading to sufficient amounts of natively refolded monodisperse membrane protein. In this connection the indicated concentrations will further optimize the results. Examples for maltosides are DDM (n-Dodecyl-β-D-maltoside, Lauryl maltoside) and TDM (Tridecyl maltoside), for bile acids are cholate and deoxycholate, for bile acids derivates are CHAPS ((3-[(3-Cholamidopropyl)-dimethylammonio]-1-propane sulfonate), CAPSO (C₃₂H₅₈N₂O₈S), BIG CHAP (N,N-bis-(3-D-Gluconamidopropyl)cholamide). Alkyl glycosides comprise all mono and disaccharides. Examples for glucamides are MEGA-8 (Octanoyl-N-methylglucamide), MEGA-9 (Nonanoyl-N-methylglucamide), MEGA-10 (Decanoyl-N-methylglucamide), HEGA (Decanoyl-N-hydroxyethylglucamide). Examples for saccharide fatty acid esters are sucrose monododecanate, Tx100 (Triton X) 100c, OG (Octyl glycoside), OTG (Octyl thioglycoside), C8E5 (Pentaethylenglycol octyl ether), C12E9 (POE 9 dodecyl ether), CYMAL_{®}-5 (5-Cyclohexyl-1-pentyl-®-D-maltoside), CYMAL_{®}-6 (6-Cyclohexyl-1-hexyl-®-D-maltoside), CYMAL_{®}-7 (7-Cyclohexyl-1-heptyl-®-D-maltoside), C12 DAO (Dodecyl dimethyl amino N-oxide), C10 DAO (DDA; Decyl dimethyl amino N-oxide) and Anzergent 3-14 (Tetradaecyl-N,N-dimethyl-3-ammonio-1-propanesulfonate.

Within the frame of the new method it is preferred if in step (b) said exchange is carried out via a chromographic method, preferably via the use of a nickel-NTA column, and/or of a ion exchange column, and/or of an affinity column, and/or of a metal chelate column.

By the usage of a chromatographic method, especially by using the indicated well-characterized and effectively functioning columns, the exchange of the detergents can be performed in a simple and easy-to-handle manner. After the addition of the chromographic column material to the sample solution, the unfolded solubilized membrane protein binds to that material which in turn can be pelleted via centrifugation. The supernatant, i.e. said first detergent and where applicable said lipid, can then be removed and changed for said second detergent. Alternatively, dialysis can also be used for exchanging the detergents.

According to a further development of the new method, within step (c) said second detergent is exchanged for a third detergent, wherein said exchange preferably is also carried out via a chromographic method, preferably by using a nickel-NTA column, and/or an ion exchange column, and/or an affinity column, and/or a metal chelate column, and/or a Superdex 200 column. That third detergent is preferably selected from the group consisting of: maltosides; alkyl phosphocholines having a chain length of C8 to C16; bile acids and derivatives; alkyl-N,N-dimethyl glycin (alkyl=C8 to C16); alkyl glycosides (alkyl=C5 to C12); glucamides; saccharide fatty acid esters.

Depending on the individual membrane protein it might be advantageous to incubate the refolded protein in the presence of a third detergent. The inventors have realized that the indicated detergents are particularly suited. The addition of the third detergent favors the growing of possibly wanted membrane protein crystals, since it provides an advantageous environment therefor.

Another object of the present invention relates to a method for preparing a crystalline form of a recombinantly expressed or chemically synthesized eukaryotic membrane protein, preferably selected from the group consisting of: receptors, preferably from the family of G-protein-coupled receptors, ion channels, transport proteins, as well as partial sequences, homologous sequences, mutated sequences and derived sequences, recombinant forms of aforementioned group members; comprising the steps of: (a) providing a solution of said membrane protein in monodisperse form, and (b) incubating the solution for growing of membrane protein crystals, wherein step (a) is performed according to the afore-mentioned new method. Preferably, transition from step (a) to step (b) occurs without interposition of a separation step for separating of protein folded into its native or active form, from protein not folded into its native or active form.

From a skilled person's view it was totally surprising and against the current knowledge on the field of protein crystallization that just by performing the further above-described new method such a homogenous and monodisperse solution of membrane protein refolded into its native or active form can be obtained which in turn can directly be subjected to the crystallization procedure. Quite the contrary, one would expect that first it would be compulsory to screen for correctly folded, i.e. active membrane proteins which on the other hand are to be separated from misfolded proteins, and that only then the crystallization procedure could be initiated.

With that afore-mentioned method it is preferred if in step (a) an accessory agent is added to said solution, preferably said agent is selected from the group consisting of: proteins including ligands of membrane receptors, receptors, peptides, antibodies, haptens; nucleic acids including aptamers; organic compounds including ligands of membrane receptors, lipids, sugars; anorganic compounds; drugs; prodrugs.

This measure has the advantage that herewith a so-called "co-crystallization" is enabled, by which the structure of a complex of, for example, a receptor and its ligand can be analysed. In this case the ligand represents the accessory agent and can be the naturally occurring ligand, a modified ligand having e.g. a higher affinity to the receptor, a drug, etc. As a result thereof, the binding position of the ligand may be found out and the ligand could be modified what concerns its characteristics, e.g. its affinity to the receptor.

Furthermore, in many cases the stability of the membrane protein is increased by the addition of an accessory agent, e.g. an antibody, so that the growing of membrane protein crystals herewith is facilitated or even just enabled.

According to a preferred development of this method, step (b) is performed according to standard crystallization screenings by "hanging drop" or/and "sitting drop" vapor diffusion, or/and micro batch, or/and micro dialysis, or/and free interface diffusion technique, said standard crystallization screenings are preferably selected from the group consisting of: Hampton Research Crystal screens, Molecular Dimensions screens, Emerald Biostructures screens, and Jena BioScience screens.

The inventors have realized that for obtaining membrane protein crystals currently used standard crystallization procedures can be applied. Especially the listed methods yield good results in connection with the new method. This finding is advantageous since one of the main problems in the area of protein crystallization so far concerns the establishment of a crystallization protocol individually adapted to the protein of interest. This means a very time-consuming and hardly to automatize approach. Contrariwise, the invention avoids the workout of such an individually made protocol since the current screening methods are well suited.

In connection with the standard screenings it is preferred if the "sitting" or "hanging drop" consisting of about 200 nl of membrane protein solution having a concentration of about 1-100 mg/ml, preferably 10 mg/ml of protein, and of about 1 nl-10 ml, preferably 200 nl of precipitant solution, and wherein the reservoir containing about 10 µl-100 ml, preferably 100 µl of precipitant solution.

Within that indicated ranges, as the inventors have recognized, the conditions for well formed membrane protein crystals are particularly optimal. The volumes and concentrations of the solutions are herewith in good coordination.

Furthermore it is preferred if said precipitant solution has a pH value of about pH 6,5-10 and comprises about 0-0,5 M, preferably 0,1 M Tris/HCl and/or Hepes/NaOH and/or NaK phosphate at that given pH value; about 5-40 % (w/v) of a polyethylene glycol (PEG) and/or polyethylene glycol mono methylether (PEG MME) with a molecular weight of about 1.000-10.000, preferably 2.000-6.000, more preferably 4.000.

By this measure optimized conditions for growing of membrane protein crystals regarding crystal size and diffraction quality are provided.

By this method a crystalline form of a recombinantly expressed or chemically synthesized eukaryotic membrane protein can be produced, preferably selected from the group consisting of: receptors, preferably from the family of G-protein-coupled receptors, ion channels, transport proteins, as well as partial sequences, homologous sequences, mutated sequences and derived sequences of aforementioned group members.

The inventors have succeeded for the first time in producing recombinantly expressed or chemically synthesized membrane proteins in crystalline form. As explained at the outset the newly provided protein crystals are very important tools for developing new drugs, e.g. by means of *in silico*-screening.

By this before-mentioned method according to the invention crystallized membrane proteins can be produced which originally have been recombinantly produced, e.g. by the usage of a yeast, CHO or insect cell expression system, and which therefore have been directly provided to the crystallization procedure in active or native form, i.e. the performance of an additional refolding step for providing active and correctly refolded protein had not been necessary. It shall be understood that also classic bacterial, e.g. *E. coli* expression systems can be used in order to provide sufficient amounts of recombinant membrane protein.

Up to now, there have been no examples in the art for successful preparation of membrane protein crystals starting from recombinantly produced membrane protein. However, crystals made of recombinant proteins have several advantages. Firstly, these kind of proteins can be provided in large amounts, e.g. contrary to the method described by Okada et al., loc.cit., where it is required to isolate the GPCRs from biological membranes. Furthermore, by the usage of recombinant expression systems one can also obtain seleno-variants of membrane proteins. In view of the crystallization procedure the structure determination, e.g. by multiple wavelength anomalous dispersion (HAD), is greatly facilitated and accelerated. Moreover, the data quality is improved compared to traditional methods, e.g. multiple isomorphous replacement (MIR).

The inventors have succeeded in developing the aforedescribed method which concerns a simple manageable approach by means of which large amounts of the listed membrane proteins for a subsequent crystallization procedure can be produced. Furthermore, by the reliable provision of correctly refolded and monomeric protein the actual crystallization procedure can even be performed according to standard protocols, which is why costly and time-consuming tests for developing suitable crystallization conditions are no longer necessary. Such crystallization methods can then be applied to membrane proteins produced directly in its active form, i.e. without requiring refolding steps.

By means of the method according to the invention also a crystalline form of a complex of a recombinantly expressed, or chemically synthesized eukaryotic membrane protein can be produced, preferably selected from the group consisting of: receptors, preferably from the family of G-protein-coupled receptors, ion channels, transport proteins, as well as partial sequences, homologous sequences, mutated sequences and derived sequences of aforementioned group members, and of an accessory agent, whereby it is preferred if said complex is prepared according to the preferred embodiment of above-mentioned method concerning the addition of an accessory agent.

As described above, the crystalline form of such a complex is e.g. very useful in order to develop new ligands of receptors having modified characteristics compared to naturally occurring ligands, for example being utilizable as drugs.

Further advantages and variations will ensue from the embodiments.

### Example 1: Production of an expression vector with cDNA for membrane protein

DNA sequences for several membrane proteins are listed in the EMBL database, in most cases, they do not have introns. With the help of primers, the required DNA can be produced via PCR from genomic DNA or via RT-PCR from mRNA.

This DNA is then cloned into an expression vector, which was constructed for the expression of a fusion protein. The tag part protein can be e.g. an histidine tail (his), as described in the art, e.g. in Sambrook, J. and Russell D.W. (2001), "Molecular cloning - A Laboratory Manual", Cold Spring Harbor Laboratory Press, New York, which is herewith incorporated by reference.

Vectors for the expression of sphingosin 1 phosphate receptor (gpr3) and of cannabinoid receptor 1 (CB1) as histidine-tagged glutathione-S-transferase (GST) fusion proteins were produced. The expression vectors were transformed into a cell line which expressed the fusion proteins. The proteins are, in this procedure, not incorporated into the membrane, but exist at least partly aggregated in form of inclusion bodies in cytoplasm and are, thus, not correctly folded.

### Example 2: Expression of recombinant membrane protein in the form of inclusion bodies

The cDNAs of above mentioned membrane proteins were each, in-frame, inserted into the vector pGEX2a-c-His. This vector contains downstream of the Tac-promotor the sequence encoding glutathione-S-transferase and a subsequent thrombin cleavage site, followed by a polylinker sequence and, finally, six histidine codons and a stop codon.

The vectors were transformed into *E*. *coli* strains derived from strain K12, e.g. BL21 or BLR. The protein expression was induced by adding IPTG, and the cells were harvested after further three hours. After lysozyme treatment and homogenization by sonification, the membranes and inclusion bodies were separated from the soluble proteins by centrifugation.

### Example 3: Solubilization of the inclusion bodies and thrombin cleavage

For each membrane protein 50 ml of inclusion bodies were centrifuged for 10 min, at 4°C at maximum speed. Each pellet was resuspended in 450 ml of the following buffer: 25 mM Tris/HCl pH 8,5, 250 mM NaCl, 1 mM DTT, and put on ice for 15 min.

Subsequently, each ice cold sample was subjected to sonification for 3 min at 50% "duty cycle" and 80% power in a Bandelin Sonoplus microsonicator using a rosette cell. It has to be ensured that the sample will be kept cool.

Afterwards 50 ml of following first detergent and lipid containing buffer solution was added to each sample: 10% L-lauroyl-sarcosine (LS) in TBS pH 8,5 containing 0,1 mg/ml brain polar lipid extract (Aranti Polar Lipids Inc., Alabaster, USA; Cat. No. 14110). The membrane protein was herewith solubilized.

To each sample 12.500 U thrombin was added, in order to cleave the GST fusion part. Each sample was slowly stirred overnight at 20°C. Afterwards, the membrane protein was solubilized. The sample was centrifuged for 20 min at ≥ 20.000 g at 4°C.

### Example 4: Inducing refolding of membrane protein

25 ml of NiNTA column material equillibrated in 50 mM Hepes/NaOH pH 7,5, 250 mM NaCl and 1% LS was added to 500 ml of solubilized membrane protein, e.g. of gpr3 or CB1. The sample was incubated for 30 to 60 min, afterwards the column material comprising the immobilized membrane protein was transferred either into an empty column or an XK26/30 column (Amersham, Buckinghamshire, UK). The following steps were performed at 4°C, i.e. all buffers used were stored on ice.

The column was washed with 10 column volumes (CV) of 50 mM Hepes/NaOH pH 7,5, 250 mM NaCl, 0,1 mg/ml brain polar liquid extract, 1 mM GSH, 1 mM GSSG, 1% LS; followed by washing with 10 CV of 50 mM Hepes/NaOH pH 7,5, 250 mM NaCl, 1% FOS-choline-14 (C14, N-tetradecylphosphocholine; second detergent).

Elution was performed using 3 CV of elution buffer, i.e. 50 mM Hepes/NaOH pH 7,5, 500 mM NaCl, 300 mM imidazole (pH 7,0), 0,1% C14. Therefor the column was incubated with the incubation buffer for 15 min at room temperature. Subsequently, fractions of 8 ml each were eluted. Aliquots of solubilized membrane protein, flow through, wash and elution fractions were separated by SDS PAGE followed by Coomassie blue staining in order to examine purity and yield.

The first three elution fractions were pooled. A typical yield is about 22 mg of protein, i.e. of gpr3 or CB1, concentrated at 1,45 mg/ml. The solution was concentrated to 10 to 15 mg/ml using an Amicon Ultra 15 ml 30 kD ultrafiltration concentrator.

### Example 5: Size exclusion chromatography

### Sphingosin 1 phosphate receptor (gpr3)

A Superdex 200 10/300 GL column (Amersham Biosciences, Buckinghamshire, UK) was equillibrated using 1,5 CV of 20 mM Hepes/NaOH pH 7,0, 200 mM NaCl, 0,1% C14. 8 times 100 µl of concentrated protein solution, i.e. about 0,8 mg, were added to the column. Elution was performed using 1,5 CV of above mentioned elution buffer. Fractions of 200 µl each were collected. The fractions were analyzed by means UV absorption at 280 nm and SDS PAGE followed by Coomassie blue staining.

Alternatively, a Superdex 200 26/60 prep grade column was used. In this case, approximately 50 mg of protein were added to the column. Fractions of 5 ml each were collected.

Monomeric fractions were pooled. The pooled sample was concentrated to 10 mg/ml using an Amicon Ultra 10 ml 30 kDa device. A typical yield is 3 mg protein. In order to confirm whether the sample is purified, i.e. consisting exclusively of monomeric membrane protein, 10 µl of concentrated protein solution was subjected to an analytic gel filtration using a Superdex 200 10/300 GL column, 20 mM Hepes/NaOH pH 7,0, 200 mM NaCl, 0,1% C14.

Alternatively, the second detergent C14 was changed for a third detergent by performing the following procedure: A Superdex 200 10/300 GL column was equilibrated using 1,5 CV of 20 mM Hepes/NaOH pH 7,0, 200 mM NaCl, and third detergent (e.g. 0,01% FOS-choline-16, or 0,01% tetradecylmaltoside). 1 mg protein of each sample was subjected to a size exclusion filtration as described before. The monomeric fractions were pooled, the solution was concentrated to 10 mg/ml using an Amicon Ultra 15 ml 30 kDa device.

### Cannabinoid receptor (CB1)

A Superdex 200 26/60 prep grade column was equilibrated using 1,5 CV of 20 mM Hepes/NaOH pH 7,0, 200 mM NaCl, 0,1% C14. 5 ml of concentrated protein solution, i.e. about 50 mg, were applied to the column. Elution was performed using 1,2 CV of above mentioned elution buffer. Fractions of 5 ml each were collected.

Monomeric fractions were pooled. The pooled sample was concentrated to 10 mg/ml using a Millipore ULTRA 100 kDa. The pooled sample was checked via a Superdex 200 HR 10/30 column using 20 mM Hepes/NaOH pH 7,0, 200 mM NaCl, 0,1% C14.

Alternatively, a Superdex 200 10/300 GL column was used. In this case, approximately 1-2 mg of protein were applied in a volume of 100 µl to the column. Fractions of 0,2 ml each were collected.

Monomeric fractions were pooled. The pooled sample was concentrated to 10 mg/ml using an Amicon Ultra 15 ml 100 kDa device. A typical yield is 5-15 mg protein. In order to confirm whether the sample is homogenous, i.e. consisting exclusively of monomeric membrane protein, 10 µl of concentrated protein solution was subjected to an analytic gel filtration using a Superdex 200 10/300 GL column with 20 mM Hepes/NaOH pH 7,0, 200 mM NaCl, 0,1 % C14 as running buffer.

Alternatively, the second detergent C14 was exchanged for a third detergent by performing the following procedure: A Superdex 200 26/60 prep grade column was equilibrated using 1,5 CV of 20 mM Hepes/NaOH, pH 7,0, 200 mM NaCl, and a third detergent (e.g. 0,01% FOS-choline-16, 0,01 % tetradecylmaltoside, or 0,1 % lauryldimethylamine N-oxide (LDAO)). 1 mg protein was subjected to a size exclusion filtration as described before. The monomeric fractions were pooled, the solution was concentrated to 10 mg/ml using an Amicon Ultra 15 ml 100 kDa device.

### Example 6: Crystallization set-up

### Sphingosin 1 phosphate receptor (gpr3)

The crystallization was performed according to "sitting drop" vapour diffusion technique. Therefor a CrystalQuick 288 plate with circular wells (Greiner, Germany) was used.

The reservoir solution was 0,1 M Tris/HCl, pH 8,5; 24% polyethyleneglycol (PEG) 4000.

Each drop was consisting of 200 nl protein solution of example 5, and of 200 nl reservoir solution.

The screening for crystals was performed according to standard screening procedures, e.g. sparse matrix sampling technology. Corresponding commercialized screens can be found at Hampton Research Inc., Aliso Viejo, USA (http://www.hamptonresearch.com/hrproducts/xtalscreens.html;
http://www.hamptonresearch.com/).

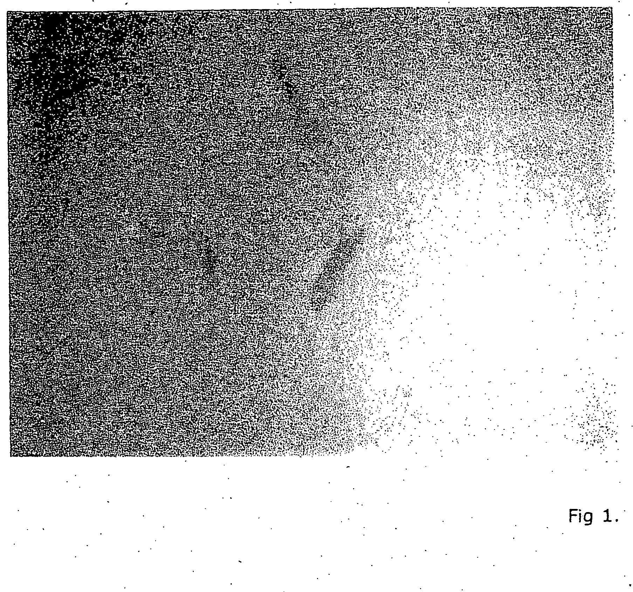

Crystals have been developed after 10 days as shown in Fig. 1.

### Cannabinoid receptor 1 (CB1)

The crystallisation was performed using the "sitting drop" vapour diffusion technique. The screening for crystals was conducted using commercially available sparse matrix screens. Corresponding screens can be found at Hampton Research Inc., Aliso Viejo, USA
(http://www.hamptonresarch.com/hrproducts/xtalscreens.html;
http://www.hamptonresearch.com/).

The reservoir solution was 0,1 M Hepes/NaOH, pH 7,0, 40 % polyethyleneglycol monomethylether (PEGMME).

Each drop was consisting of 100-200 nl protein solution of example 5, and of 100-200 nl reservoir solution.

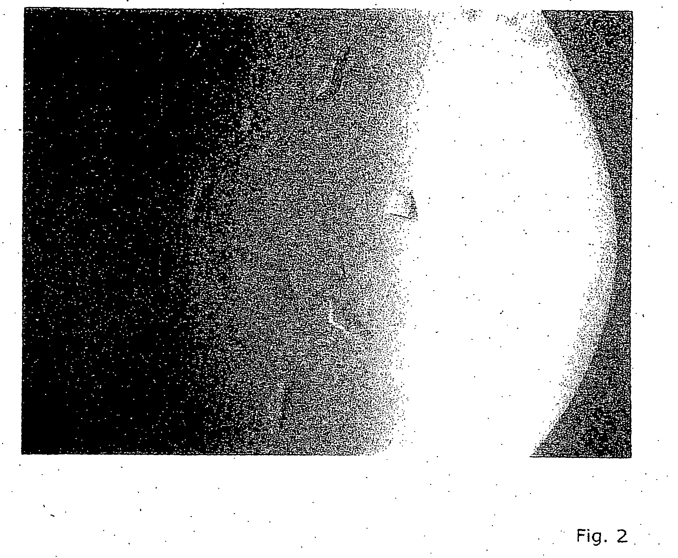

Crystals grew after 10-14 days at 18°C as shown in Fig. 2.

## Claims

1. A method for preparing a solution of a refolded, recombinantly expressed or chemically synthesized eukaryotic membrane protein in monodisperse form, consisting of the steps:
(a) providing said membrane protein in non-correctly folded form and solubilized in a first detergent,
(b) inducing refolding of said membrane protein into its native or active form, and
(c) performing a size exclusion chromatography on said solution of refolded membrane protein.

2. The method of claim 1, comprising between steps (a) and (b) the further step of:
(a') adding a lipid to said membrane protein solution.

3. The method of claim 1 or 2, **characterized in that** step (b) comprises the step of:
(b') exchanging said first detergent for a second detergent.

4. The method of claim 1 or 2, **characterized in that** step (b) comprises the step of:
(b'') diluting said first detergent to an adequately low concentration.

5. The method of any of claims 1 to 4, **characterized in that** said membrane protein is selected from the group consisting of: receptors, preferably from the family of G-protein-coupled receptors, ion channels, transport proteins as well as partial sequences, homologous sequences, mutated sequences and derived sequences of aforementioned group members.

6. The method of any of claims 1 to 5, **characterized in that** said membrane protein is a mammalian, preferably a human protein.

7. The method of any of claims 1 to 6, **characterized in that** said membrane protein is a histidine-tagged fusion protein.

8. The method of any of claims 1 to 7, **characterized in that** said membrane protein is provided in form of inclusion bodies, preferably as a bacterial expressed protein, more preferably as an *E. coli* expressed protein.

9. The method of any of claims 1 to 7, **characterized in that** said membrane protein is provided in form of inclusion bodies being synthesized by means of a cell-free expression system, preferably of the Rapid Translation System (RTS).

10. The method of any of claims 2 to 9, **characterized in that** said lipid is selected from the group consisting of: naturally extracted phospholipids and synthetic phospholipids; especially brain polar lipid extract, phosphatidyl choline, phosphatidyl ethanolamine, cholesterol, phospholipid, ergosterol, asolectin, sphingomyelin, DOPA.

11. The method of any of claims 2 to 10, **characterized in that** said lipid is added to a final concentration of about 0,01 to 5 mg/ml, preferably of about 0,05 to 2 mg/ml, more preferably of about 1 mg/ml.

12. The method of any of claims 1 to 11, **characterized in that** said first detergent is selected from the group consisting of: FOS-choline-8, FOS-choline-9, FOS-choline-10, FOS-choline-11, FOS-choline-12, FOS-choline-13, FOS-choline-14, FOS-choline-15, FOS-choline-16, and N-laroyl-sarcosine.

13. The method of any of claims 1 to 12, **characterized in that** said first detergent is provided in a final concentration of about 0,1 1 to 5, preferably of about 0,5 to 4, more preferably of about 1 % (w/v).

14. The method of any of claims 1 to 13, **characterized in that** in step (b) additionally SDS and/or urea is added to the membrane protein solution.

15. The method of any of claims 3 to 14, **characterized in that** said second detergent is selected from the group consisting of charged and uncharged detergents, preferably from the group consisting of: maltosides; alkyl phosphocholines having a chain length of C8 to C16; bile acids and derivatives; alkyl-N,N-dimethyl glycine (alkyl=C8 to C16); alkyl glycosides (alkyl=C5 to C12); glucamides; saccharide fatty acid esters.

16. The method of any of claims 3, 5 to 15, **characterized in that** said second detergent is provided in a final concentration of about 0,01 to 5, preferably of about 0,05 to 1, more preferably of about 0,1 % (w/v).

17. The method of any of claims 3 to 16, **characterized in that** in step (b') said exchange is carried out via a chromographic method, preferably via the use of a nickel-NTA column, and/or of a ion exchange column, and/or of an affinity column, and/or of a metal chelate column.

18. The method of any of claims 3 to 17, **characterized in that** within step (c) said second detergent is exchanged for a third detergent.

19. The method of claim 18, **characterized in that** said exchange is carried out via a chromatographic method, preferably via the use of a nickel-NTA column, and/or of an ion exchange column, and/or of an affinity column, and/or of a metal chelate column, and/or a Superdex 200 column.

20. The method of claim 18 or 19, **characterized in that** said third detergent is selected from the group consisting of: maltosides; alkyl phosphocholines having a chain length of C8 to C16; bile acids and derivatives; alkyl-N,N-dimethyl glycine (alkyl=C8 to C16); alkyl glycosides (alkyl=C5 to C12); glucamides; saccharide fatty acid esters.

21. A method for preparing a crystalline form of a recombinantly expressed, or chemically synthesized eukaryotic membrane protein, preferably selected from the group consisting of: receptors, preferably from the family of G-protein-coupled receptors, ion channels, transport proteins, as well as partial sequences, homologous sequences, mutated sequences and derived sequences, recombinant forms of aforementioned group members; comprising the steps of:
(a) Providing a solution of said membrane protein in monodisperse form, and
(b) Incubating the solution for growing of membrane protein crystals,
**characterized in that** step (a) is performed according to the method of any of claims 1 to 20.

22. The method of claim 21, **characterized in that** transition from step (a) to step (b) occurs without interposition of a separation step for separating of protein folded into its native or active form, from protein not folded into its native or active form.

23. The method of claim 21 or 22, **characterized in that** in step (a) an accessory agent is added to said solution, preferably said agent is selected from the group consisting of: proteins including ligands of membrane receptors, receptors, peptides, antibodies, haptens; nucleic acids including aptamers; organic compounds including ligands of membrane receptors, lipids, sugars; anorganic compounds; drugs; prodrugs.

24. The method of any of claims 21 to 23, **characterized in that** step (b) is performed according to standard crystallization screenings by "hanging drop" or/and "sitting drop" vapor diffusion, or/and micro batch, or/and micro dialysis, or/and free interface diffusion technique, said standard crystallization screenings are preferably selected from the group consisting of: Hampton Research Crystal screens, Molecular Dimensions screens, Emerald Biostructures screens, and Jena Bioscience screens.

25. The method of claim 24, **characterized in that** the "sitting" or "hanging drop" consisting of about 200 nl of membrane protein solution having a concentration of about 1-100 mg/ml, preferably 10 mg/ml of protein, and of about 1 nl-10 ml, preferably 200 nl of precipitant solution, and **characterized in that** the reservoir containing about 10 µl-100 ml, preferably 100 µl of precipitant solution.

26. The method of claim 25, **characterized in that** said precipitant solution has a pH value of about pH 6,5-10 and comprises about 0-0,5 M, preferably 0,1 M Tris/HCl and/or Hepes/NaOH and/or NaK phosphate at that given pH value; about 5-40 % (w/v) of a polyethylene glycol (PEG) and/or polyethylene glycol mono methylether (PEG MME) with a molecular weight of about 1.000-10.000, preferably 2.000-6.000, more preferably 4.000.

## Patentansprüche

1. Verfahren zur Herstellung einer Lösung eines rückgefalteten, rekombinant exprimierten oder chemisch synthetisierten, eukaryotischen Membranproteins in monodisperser Form, das aus den folgenden Schritten besteht:
(a) Bereitstellen des Membranproteins in nicht-korrekt gefalteter Form und gelöst in einem ersten Detergens,
(b) Induzieren der Rückfalten des Membranproteins in dessen native oder aktive Form, und
(c) Durchführen einer Größenausschlusschromatographie an der Lösung des rückgefalteten Membranproteins.

2. Verfahren nach Anspruch 1, das zwischen den Schritten (a) und (b) den folgenden weiteren Schritt aufweist:
(a') Zugeben eines Lipides zu der Membranproteinlösung.

3. Verfahren nach Anspruch 1 oder 2, **dadurch gekennzeichnet, dass** Schritt (b) den folgenden Schritt aufweist:
(b') Austauschen des ersten Detergens gegen ein zweites Detergens.

4. Verfahren nach Anspruch 1 oder 2, **dadurch gekennzeichnet, dass** Schritt (b) den folgenden Schritt aufweist:
(b") Verdünnen des ersten Detergens auf eine geeignete niedrige Konzentration.

5. Verfahren nach einem der Ansprüche 1 bis 4, **dadurch gekennzeichnet, dass** das Membranprotein ausgewählt ist aus der Gruppe bestehend aus: Rezeptoren, vorzugsweise aus der Familie der G-Protein-gekoppelten Rezeptoren, Ionenkanälen, Transportproteinen sowie Teilsequenzen, homologen Sequenzen, mutierten Sequenzen und abgeleiteten Sequenzen der zuvor erwähnten Gruppenmitglieder.

6. Verfahren nach einem der Ansprüche 1 bis 5, **dadurch gekennzeichnet, dass** das Membranprotein ein Säugetier-, vorzugsweise ein humanes Protein ist.

7. Verfahren nach einem der Ansprüche 1 bis 6, **dadurch gekennzeichnet, dass** das Membranprotein ein mit einem Histidin-Tag versehenes Fusionsprotein ist.

8. Verfahren nach einem der Ansprüche 1 bis 7, **dadurch gekennzeichnet, dass** das Membranprotein in Form von Einschlusskörpern bereitgestellt wird, vorzugsweise als ein bakteriell exprimiertes Protein, weiter bevorzugt als ein in *E*. *coli* exprimiertes Protein.

9. Verfahren nach einem der Ansprüche 1 bis 7, **dadurch gekennzeichnet, dass** das Membranprotein in Form von Einschlusskörpern bereitgestellt wird, die mittels eines zellfreien Expressionssystems, vorzugsweise des Rapid Translation Systems (RTS), synthetisiert wurden.

10. Verfahren nach einem der Ansprüche 2 bis 9, **dadurch gekennzeichnet, dass** das Lipid ausgewählt ist aus der Gruppe bestehend aus: Natürlich extrahierten Phospholipiden und synthetischen Phospholipiden; insbesondere polarer Lipidextrakt aus dem Gehirn, Phosphatidylcholin, Phosphatidylethanolamin, Cholesterol, Phospholipid, Ergosterol, Asolectin, Sphingomyelin, DOPA.

11. Verfahren nach einem der Ansprüche 2 bis 10, **dadurch gekennzeichnet, dass** das Lipid in einer Endkonzentration von etwa 0,01 bis 5 mg/ml, vorzugsweise von etwa 0,05 bis 2 mg/ml, weiter bevorzugt von etwa 1 mg/ml hinzugegeben wird.

12. Verfahren nach einem der Ansprüche 1 bis 10, **dadurch gekennzeichnet, dass** das erste Detergens ausgewählt ist aus der Gruppe bestehend aus: FOS-Cholin-8, FOS-Cholin-9, FOS-Cholin-10, FOS-Cholin-11, FOS-Cholin-12, FOS-Cholin-13, FOS-Cholin-14, FOS-Cholin-15, FOS-Cholin-16 und N-Laroyl-Sarcosin.

13. Verfahren nach einem der Ansprüche 1 bis 12, **dadurch gekennzeichnet, dass** das erste Detergens bereitgestellt wird in einer Endkonzentration von etwa 0,1 bis 5, vorzugweise von etwa 0,5 bis 5, weiter bevorzugt von etwa 1% (w/v).

14. Verfahren nach einem der Ansprüche 1 bis 13, **dadurch gekennzeichnet, dass** in Schritt (b) zusätzlich SDS und/oder Harnstoff zu der Membranproteinlösung hinzugegeben wird.

15. Verfahren nach einem der Ansprüche 3 bis 14, **dadurch gekennzeichnet, dass** das zweite Detergens ausgewählt ist aus der Gruppe bestehend aus geladenen und ungeladenen Detergenzien, vorzugsweise aus der Gruppe bestehend aus: Maltosiden; Alkylphosphocholinen mit einer Kettenlänge von C8 bis C16; Gallensäuren und Derivaten; Alkyl-N,N-Dimethylglyzin (Alkyl=C8 bis C16); Alkylglykosiden (Alkyl=C5 bis C12); Glukamiden; Saccharidfettsäureestern.

16. Verfahren nach einem der Ansprüche 3, 5 bis 15, **dadurch gekennzeichnet, dass** das zweite Detergens bereitgestellt wird in einer Endkonzentration von etwa 0,01 bis 5, vorzugsweise von etwa 0,05 bis 1, weiter bevorzugt von etwa 0,1% (w/v).

17. Verfahren nach einem der Ansprüche 3 bis 16, **dadurch gekennzeichnet, dass** in Schritt (b') der Austausch über ein chromatographisches Verfahren durchgeführt wird, vorzugsweise über die Verwendung einer Nickel-NTA-Säule, und/oder einer Ionenaustauschersäule, und/oder einer Affinitätssäule und/oder einer Metallchelatsäule.

18. Verfahren nach einem der Ansprüche 3 bis 17, **dadurch gekennzeichnet, dass** in Schritt c) das zweite Detergens gegen ein drittes Detergens ausgetauscht wird.

19. Verfahren nach Anspruch 18, **dadurch gekennzeichnet, dass** der Austausch über ein chromatographisches Verfahren durchgeführt wird, vorzugsweise über die Verwendung einer Nickel-NTA-Säule, und/oder einer Ionenaustauschersäule, und/oder einer Affinitätssäule und/oder einer Metallchelatsäule, und/oder einer Säule Superdex 200.

20. Verfahren nach Anspruch 18 oder 19, **dadurch gekennzeichnet, dass** das dritte Detergens ausgewählt ist aus der Gruppe bestehend aus: Maltosiden; Alkylphosphocholinen mit einer Kettenlänge von C8 bis C16, Gallensäuren und Derivaten; Alkyl-N,N-Dimethylglyzin (Alkyl=C8 bis C16); Alkylglykosiden (Alkyl=C5 bis C12); Glukamiden; Saccharidfettsäureestern.

21. Verfahren zur Herstellung einer kristallinen Form eines rekombinant exprimierten, oder chemisch synthetisierten eukaryotischen Membranproteins, vorzugsweise ausgewählt aus der Gruppe bestehend aus: Rezeptoren, vorzugsweise aus der Familie der G-Protein-gekoppelten Rezeptoren, Ionenkanälen, Transportproteinen, sowie Teilsequenzen, homologen Sequenzen, mutierten Sequenzen und abgeleiteten Sequenzen, rekombinanten Formen der zuvor erwähnten Gruppenmitglieder; das folgende Schritte aufweist:
(a) Bereitstellen einer Lösung des Membranproteins in monodisperser Form, und
(b) Inkubieren der Lösung, um Kristalle des Membranproteins entwickeln zu lassen;
**dadurch gekennzeichnet, dass** Schritt (a) durchgeführt wird gemäß dem Verfahren nach einem der Ansprüche 1 bis 20.

22. Verfahren nach Anspruch 21, **dadurch gekennzeichnet, dass** der Übergang von Schritt (a) nach Schritt (b) ohne die Zwischenschaltung eines Schrittes zur Separierung von Proteinen, die sich in deren native oder aktive Form gefaltet haben, von Proteinen, die sich nicht in deren native oder aktive Form gefaltet haben.

23. Verfahren nach Anspruch 21 oder 22, **dadurch gekennzeichnet, dass** in Schritt (a) ein Hilfsstoff zu der Lösung hinzugegeben wird, wobei der Stoff vorzugsweise ausgewählt ist aus der Gruppe bestehend aus: Proteinen einschließlich Liganden von Membranrezeptoren, Rezeptoren, Peptiden, Antikörpern, Haptenen; Nukleinsäuren einschließlich Aptamere; organische Verbindungen einschließlich Liganden von Membranrezeptoren, Lipiden, Zuckern; anorganischen Verbindungen; Pharmaka, Pro-Pharmaka.

24. Verfahren nach einem der Ansprüche 21 bis 23, **dadurch gekennzeichnet, dass** Schritt (b) durchgeführt wird gemäß standardisierten Kristallisierungsselektionen nach den Dampfdiffusionsverfahren des "hängenden Tropfens" und/oder "sitzenden Tropfens", und/oder Micro Batch, und/oder Microdialyse, und/oder einer Technik der freien Grenzflächendiffusion, wobei die standardisierten Kristallisierungsselektionen vorzugsweise ausgewählt sind aus der Gruppe bestehend aus: Hampton-Research-Crystal-Selektionen, Molecular-Dimensions-Selektionen, Emerald-Biostructures-Selektionen, und Jena BioScience-Selektionen.

25. Verfahren nach Anspruch 24, **dadurch gekennzeichnet, dass** der "sitzende" oder "hängende Tropfen" aus etwa 200 nl der Lösung mit dem Membranprotein besteht, die eine Konzentration von etwa 1-100 mg/ml, vorzugsweise 10 mg/ml Protein, und etwa 1 nl-10 ml, vorzugsweise 200 nl Lösung des Präzipitats aufweist, und **dadurch gekennzeichnet ist, dass** das Reservoir etwa 10 µl-100 ml, vorzugsweise 100 µl der Lösung mit Präzipitat enthält.

26. Verfahren nach Anspruch 25, **dadurch gekennzeichnet, dass** die Lösung mit Präzipitat einen pH-Wert von etwa pH 6,5-10 aufweist und bei diesem pH-Wert etwa 0-0,5 M, vorzugsweise 0,1 M Tris/HCl und/oder Hepes/NaOH und/oder NaK-Phosphat; etwa 5-40 % (w/v) eines Polyethylenglykols (PEG) und/oder Polyethylenglykol Monoether (PEG MME) mit einem Molekulargewicht von etwa 1.000-10.000, vorzugsweise 2.000-6.000, weiter bevorzugt 4.000, aufweist.

## Revendications

1. Procédé de préparation d'une solution constituée d'une protéine de la membrane eucaryote repliée, exprimée par recombinaison ou synthétisée par voie chimique, sous forme monodispersée, constituée des étapes consistant à :
(a) fournir ladite protéine de la membrane qui présente un repliement incorrect et qui est solubilisée dans un premier détergent,
(b) induire le repliement de ladite protéine de la membrane de manière à ce qu'elle recouvre sa forme native ou active, et
(c) effectuer une chromatographie d'exclusion diffusion de ladite solution de protéine de la membrane repliée.

2. Procédé selon la revendication 1 comprenant, entre l'étape (a) et l'étape (b), une étape supplémentaire consistant à :
(a') ajouter un lipide à ladite solution de protéine de la membrane.

3. Procédé selon la revendication 1 ou 2, **caractérisé en ce que** l'étape (b) comprend l'étape consistant à :
(b') échanger ledit premier détergent avec un second détergent.

4. Procédé selon la revendication 1 ou 2, **caractérisé en ce que** l'étape (b) comprend l'étape consistant à :
(b") diluer ledit premier détergent à une concentration suffisamment faible.

5. Procédé selon l'une quelconque des revendications 1 à 4, **caractérisé en ce que** ladite protéine de la membrane est choisie dans le groupe constitué par : les récepteurs, de préférence de la famille des récepteurs couplés aux protéines G, les canaux ioniques, les protéines de transport ainsi que les séquences partielles, les séquences homologues, les séquences mutées et les séquences dérivées des éléments des groupes susmentionnés.

6. Procédé selon l'une quelconque des revendications 1 à 5, **caractérisé en ce que** ladite protéine de la membrane est une protéine de mammifère, de préférence une protéine d'humain.

7. Procédé selon l'une quelconque des revendications 1 à 6, **caractérisé en ce que** ladite protéine de la membrane est une protéine hybride marquée à l'histidine.

8. Procédé selon l'une quelconque des revendications 1 à 7, **caractérisé en ce que** ladite protéine de la membrane est fournie sous forme de corps d'inclusion, de préférence sous forme d'une protéine exprimée par une bactérie, de préférence sous forme d'une protéine exprimée par *E*. *coli.*

9. Procédé selon l'une quelconque des revendications 1 à 7, **caractérisé en ce que** ladite protéine de la membrane est fournie sous forme de corps d'inclusion qui sont synthétisés par l'intermédiaire d'un système d'expression acellulaire, de préférence le système de traduction rapide RTS.

10. Procédé selon l'une quelconque des revendications 2 à 9, **caractérisé en ce que** ledit lipide est choisi dans le groupe constitué par: les phospholipides extraits naturellement et les phospholipides synthétiques ; en particulier les extraits lipidiques polaires de cerveau, la phosphatidyl choline, la phosphatidyl éthanolamine, le cholestérol, les phospholipides, l'ergostérol, l'asolectine, la sphingomyéline, la DOPA.

11. Procédé selon l'une quelconque des revendications 2 à 10, **caractérisé en ce que** ledit lipide est ajouté à une concentration finale d'environ 0,01 à 5 mg/ml, de préférence d'environ 0,05 à 2 mg/ml, de préférence d'environ 1 mg/ml.

12. Procédé selon l'une quelconque des revendications 1 à 11, **caractérisé en ce que** ledit premier détergent est choisi dans le groupe constitué par : la FOS-choline-8, la FOS-choline-9, la FOS-choline-10, la FOS-choline-11, la FOS-choline-12, la FOS-choline-13, la FOS-choline-14, la FOS-choline-15, la FOS-choline-16 et la N-laroyl-sarcosine.

13. Procédé selon l'une quelconque des revendications 1 à 12, **caractérisé en ce que** ledit premier détergent est fourni à une concentration finale d'environ 0,1 à 5, de préférence d'environ 0,5 à 4, de préférence d'environ 1 % (p/v).

14. Procédé selon l'une quelconque des revendications 1 à 13, **caractérisé en ce qu'**à l'étape (b) une quantité supplémentaire de SDS et/ou d'urée est ajoutée à la solution de protéine de la membrane.

15. Procédé selon l'une quelconque des revendications 3 à 14, **caractérisé en ce que** ledit deuxième détergent est choisi dans le groupe constitué de détergents chargés et non chargés, de préférence dans le groupe constitué par : les maltosides ; les phosphocholines d'alkyle ayant une longueur de chaîne de C₈ à C₁₆ ; les acides biliaires et leurs dérivés ; la glycine d'alkyl-N,N-diméthyl (groupe alkyle en C₈ à C₁₆) ; les glycosides d'alkyle (groupe alkyle en C₅ à C₁₂) ; les glucamides ; les esters d'acides gras des saccharides.

16. Procédé selon l'une quelconque des revendications 3, 5 à 15, **caractérisé en ce que** ledit deuxième détergent est fourni à une concentration finale d'environ 0,01 à 5, de préférence d'environ 0,05 à 1, de préférence d'environ 0,1 % (p/v).

17. Procédé selon l'une quelconque des revendications 3 à 16, **caractérisé en ce qu'**à l'étape (b') ledit échange est effectué par une méthode chromatographique, de préférence en utilisant une colonne de nickel-NTA, et/ou une colonne échangeuse d'ions, et/ou une colonne par affinité, et/ou une colonne de chélate métal.

18. Procédé selon l'une quelconque des revendications 3 à 17, **caractérisé en ce qu'**au sein de l'étape (c), ledit deuxième détergent est échangé avec un troisième détergent.

19. Procédé selon la revendication 18, **caractérisé en ce que** ledit échange est effectué par une méthode chromatographique, de préférence en utilisant une colonne de nickel-NTA, et/ou une colonne échangeuse d'ions, et/ou une colonne par affinité, et/ou une colonne de chélate métal, et/ou une colonne Superdex 200.

20. Procédé selon la revendication 18 ou 19, **caractérisé en ce que** ledit troisième détergent est choisi dans le groupe constitué par : les maltosides ; les phosphocholines d'alkyle ayant une longueur de chaîne de C₈ à C₁₆ ; les acides biliaires et leurs dérivés ; la glycine d'alkyl-N,N-diméthyl (groupe alkyle en C₈ à C₁₆) ; les glycosides d'alkyle (groupe alkyle en C₅ à C₁₂) ; les glucamides ; les esters d'acides gras des saccharides.

21. Procédé de préparation d'une forme cristalline d'une protéine de la membrane eucaryote exprimée par recombinaison ou synthétisée par voie chimique, de préférence choisie dans le groupe constitué par : les récepteurs, de préférence de la famille des récepteurs couplés aux protéines G, les canaux ioniques, les protéines de transport ainsi que les séquences partielles, les séquences homologues, les séquences mutées et les séquences dérivées, les formes recombinées des éléments des groupes susmentionnés ; comprenant les étapes consistant à :
(a) fournir une solution de ladite protéine de la membrane sous forme monodispersée, et
(b) incuber la solution pour que les cristaux de protéine de la membrane se développent,
**caractérisé en ce que** l'étape (a) est effectuée selon le procédé selon l'une quelconque des revendications 1 à 20.

22. Procédé selon la revendication 21, **caractérisé en ce que** la transition de l'étape (a) à l'étape (b) se produit sans intercaler une étape de séparation permettant de séparer une protéine repliée dans sa forme native ou active, d'une protéine non repliée dans sa forme native ou active.

23. Procédé selon la revendication 21 ou 22, **caractérisé en ce qu'**à l'étape (a) un agent accessoire est ajouté à ladite solution, ledit agent étant de préférence choisi dans le groupe constitué par : les protéines comprenant des ligands de récepteurs membranaires, des récepteurs, des peptides, des anticorps, des haptènes ; des acides nucléiques comprenant des aptamères ; des composés organiques comprenant des ligands de récepteurs membranaires, des lipides, des sucres ; des composés anorganiques ; des médicaments ; des promédicaments.

24. Procédé selon l'une quelconque des revendications 21 à 23, **caractérisé en ce que** l'étape (b) est effectuée selon des méthodes de cristallisation standard par diffusion de vapeur en gouttes suspendues (hanging drop) ou/et en gouttes posées (sitting drop), ou/et par microcharges, et/ou par microdialyse, ou/et par diffusion d'interface libre, lesdites méthodes de cristallisation standard étant de préférence choisies dans le groupe constitué par : les systèmes de Hampton Research Crystal, les systèmes de Molecular Dimensions, les systèmes de Emerald Biostructures et les systèmes de Jena BioScience.

25. Procédé selon la revendication 24, **caractérisé en ce que** la méthode en gouttes posées (sitting drop) ou en gouttes suspendues (hanging drop) est constituée par environ 200 nl de solution de protéine de la membrane ayant une concentration d'environ 1 à 100 mg/ml, de préférence de 10 mg/ml de protéine, et par environ 1 nl à 10 ml, de préférence 200 nl de solution de précipitant, et **caractérisé en ce que** le réservoir contient environ 10 µl à 100 ml, de préférence 100 µl de solution de précipitant.

26. Procédé selon la revendication 25, **caractérisé en ce que** ladite solution de précipitant a une valeur pH d'environ 6,5 à 10 et comprend environ 0 à 0,5 M, de préférence 0,1 M de Tris/HCl et/ou d'Hepes/NaOH et/ou de phosphate NaK à cette valeur pH donnée ; environ 5 à 40 % (p/v) d'un polyéthylèneglycol (PEG) et/ou d'un polyéthylèneglycol monométhyléther (PEG/MME) avec un poids moléculaire d'environ 1 000 à 10 000, de préférence de 2 000 à 6 000, de préférence de 4 000.
